# EUROPEAN PATENT APPLICATION

(11) **EP 3 868 859 A2**
(43) Date of publication of application: **25.08.2021**
(21) Application number: 21153926.7
(22) Date of filing: 28.01.2021
(51) Int. Cl.: C12M 1/107, C05F 17/20, C12F 3/02, C12M 1/00, C12P 5/02, C12P 7/62

(54) **PLANT AND METHOD FOR THE PRODUCTION OF METHANE**

(30) Priority: 28.01.2020 IT 202000001609
(71) Applicant: SEA S.r.l., 41123 Modena (MO) (IT)
(72) Inventor: FRANCIOSI, Stefano, 41123 MODENA (IT)
(74) Representative: Biggi, Cristina

(57) **Abstract**

The patent application relates to a process for producing methane from the anaerobic fermentation of a biomass, the process comprising at least the following steps:
(a) subjecting to anaerobic digestion a quantity greater than or equal to 700,000 t/year, preferably 700,000-900,000 t/year, of biomass as such having an average value VS/Tq greater than or equal to 27.0% by weight, preferably greater than or equal to 30.0% by weight, more preferably greater than or equal to 33.0% by weight, in at least one bioreactor 2 having a total digestion volume less than or equal to 350,000 m³, preferably 150,000-350,000 m³, and producing a flow 3 of biogas comprising methane and CO₂ and a flow 4 of digestate comprising a solid phase and a liquid phase;
(b) sending the flow 3 of biogas to a refining plant 5 for selectively removing the CO₂ from the flow 3 of biogas, thus obtaining a flow 6 of CO₂ and a flow 7 of biomethane;
wherein the quantity of methane produced is greater than or equal to 70,000,000 m³/year, preferably greater than or equal to 90,000,000 m³/year.

## Description

### FIELD OF THE INVENTION

The present invention relates to a plant for the production of methane wherein the production waste and by-products are efficiently used in the plant, thus reducing the environmental impact of the entire production cycle.

### PRIOR ART

For several decades now the increasing awareness about the problems tied to the environment has given rise to a broad debate on the future of the planet; this has led, among other things, to the formulation of the concept of "sustainable development", defined as development capable of meeting the needs of present generations without compromising the ability of future generations to meet their own (Gro Harlem Brundtland;1987). Far from being a definitive condition of harmony, sustainable development is rather a process of change whereby the exploitation of resources, the direction of investments, the orientation of technological development and institutional changes are made compatible with future needs, as well as with present needs.

In order to limit the effects of climate change, priority areas of intervention have been identified on an international level; these include the energy sector, in which the priority is to reduce the use of fossil fuels, both in the production of electricity and as uncontrolled emissions of further energy sources of fossil origin, industrial processes in general, agriculture and the reuse of waste.

With reference to the energy sector, traditional non-renewable energy sources, oil and coal in particular, are not only being rapidly exhausted, but are also responsible for the high emissions of CO₂ into the air, the main cause of atmospheric pollution.

The drive towards reducing the use of fossil fuels has led to the development of so-called renewable energies, which have the characteristic of not being exhausted at the end of the cycle, in addition to being clean and economical.

In this context, the main task of the invention is to provide a process for the production of energy from renewable sources and a plant for the execution of this process. Within the scope of this task, a further object of the invention is to provide a process and a plant for the production of energy from renewable sources wherein the by-products and waste deriving from the production of energy are used within the production cycle, thus significantly reducing the environmental impact of the entire process.

### SUMMARY OF THE INVENTION

The subject matter of the present description relates to a process for producing methane from the anaerobic fermentation of a biomass as described in the independent claim 7 and in claims 8-15 dependent thereon.

Furthermore, the present description refers to a plant for producing methane from the anaerobic fermentation of a biomass as described in independent claim 1 and in the dependent claims 2-6, said plant being particularly suitable for the execution of the process according to claims 7-15.

### DESCRIPTION OF THE FIGURES

Additional features and advantages of the present solution will emerge more clearly from the following approximate and thus non-limiting description of a preferred but not exclusive embodiment of a process and a plant per the production of biomethane, wherein:
- figure 1 illustrates a block diagram of the process for obtaining methane, relating to the steps of anaerobic digestion, methanation of the residual CO₂ and purification of the flow of methane in accordance with the present description;
- figure 2 illustrates a block diagram of the process for obtaining methane according to the present invention, relating to the steps of treatment and reuse of the digestate produced by the anaerobic digestion plant.

### DETAILED DESCRIPTION OF THE INVENTION

In the context of the present description and in the appended claims, the term biomass refers to the biodegradable fraction of the products, by-products, waste and residues of biological origin originating from agriculture, forestry and connected industries, including fishing and aquaculture, as well as the biodegradable part of waste, including industrial and municipal waste of biological origin. The term vegetable biomass refers to the biodegradable fraction of the products and/or by-products and/or waste and/or residues of biological origin originating from agriculture and/or forestry and/or connected industries, a vegetable biomass consisting essentially of vegetable substances.

The term bacterial biomass refers to a biomass consisting essentially of bacteria, optionally in association with the culture medium thereof.

In the present description and in the appended claims, the term "biomass as such (Tq)" refers to a fresh biomass, upstream of any treatment that may be performed to subject the biomass itself to fermentation, including dilution with water.

The term biogas refers to a gas produced from the fermentation of a biomass. The term biomethane refers to the methane comprised in a biogas.

The term volatile solids (VS) refers to the quantity of organic matter contained in a biomass which is potentially transformable into methane.

The term dry matter (DM) refers to quantity of matter present in a biomass after the removal of the water. For the purposes of the present description and of the appended claims, the determination of dry matter is achieved by drying a sample of biomass as such weighing 0.5 kg for 2 hours at a temperature of 90°C.

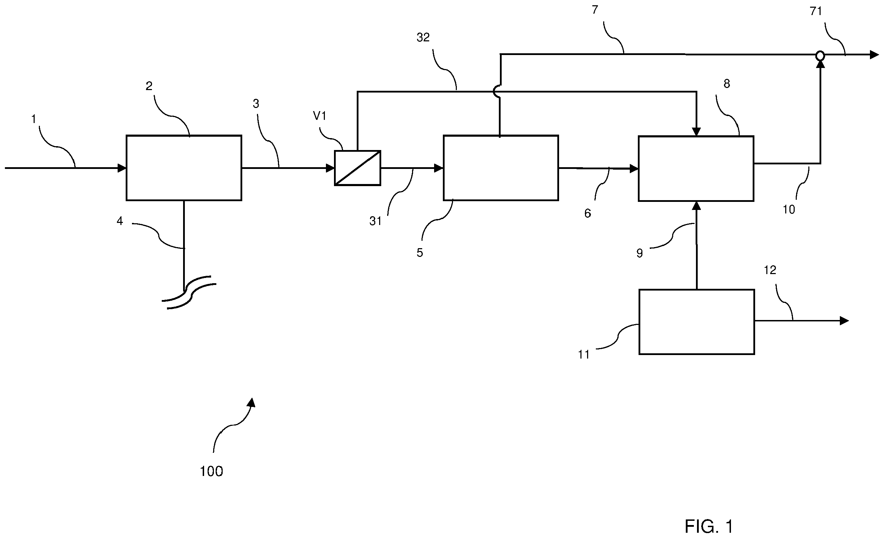

Figure 1 schematically shows the main steps of a process for the production of methane from the anaerobic fermentation of a biomass. With reference to figure 1, in a first aspect thereof, the present description refers to a process for producing methane from the anaerobic fermentation of a biomass, the process comprising at least the following steps:
(a) subjecting to anaerobic digestion a quantity greater than or equal to 700,000 t/year, preferably 700,000-900,000 t/year, of biomass as such, having a value VS/Tq greater than or equal to 27.0% by weight in at least one bioreactor 2 having a total digestion volume less than or equal to 350,000 m³, preferably 150,000-350,000 m³, and producing a flow 3 of biogas comprising biomethane and CO₂ and a flow of digestate 4 comprising a solid phase and a liquid phase;
(b) sending the flow 3 of biogas to a biogas refining plant 5 for selectively removing the CO₂ from the flow 3 of biogas, thus obtaining a flow 6 of CO₂ and a flow 7 of biomethane;
wherein the quantity of methane produced is greater than or equal to 70,000,000 m³/year, preferably greater than or equal to 90,000,000 m³/year.

The value VS/Tq represents the ratio between the volatile solids comprised in a biomass as such and the weight of the biomass as such. In one embodiment, this value is preferably greater than or equal to 30.0% by weight, more preferably greater than or equal to 33.0% by weight.

In one embodiment, the biomass as such is a vegetable biomass, preferably comprising waste and/or by-products of the production of sugar from sugar beet.

According to this embodiment, the vegetable biomass can comprise vegetable matrices with a low moisture content and vegetable matrices with a high moisture content, i.e. fresh matrices.

Vegetable matrices with a low moisture content can be accumulated in storage facilities normally used for this purpose, preferably provided with air intake systems to prevent the dissemination of odours. Before being fed to a bioreactor 2, said vegetable matrices with a low moisture content are preferably subjected to a mixing and/or crushing step.

Vegetable matrices with a high moisture content will preferably not be stored, but rather directly supplied to the bioreactor 2, optionally premixing them with vegetable matrices with a low moisture content during the mixing and/or crushing step.

The step of mixing and/or crushing the biomass preferably comprises adding water to the vegetable biomass until reaching the concentration of biomass as such which is optimal for being supplied to the bioreactor 2. The at least one bioreactor 2 can be selected in the group consisting of bioreactors of a dry, semi-dry or wet type or combinations thereof, of the type normally used in the sector and thus not further described here in detail.

The concentration of biomass as such in water in the supply of bioreactors of the dry type is generally less than or equal to 10% by weight; in the supply of bioreactors of a semi-dry type it is generally 10-25% by weight; and in the supply of bioreactors of a wet type it is generally greater than 25% by weight.

In a particularly preferred embodiment, the biomass as such comprises:
(A) 20-30% by weight of sugar beet pulp;
(B) 15-30% by weight of molasses;
(C) 50-60% by weight of sugar beet leaves and sugar beet tops,
wherein the quantities (A), (B) and (C) refer to the total weight of the biomass as such, i.e. to the weight of (A)+(B)+(C).

According to this embodiment, the process is particularly efficient and the quantity of methane produced is particularly high.

Advantageously, the biomass as such has at least one of the following properties:
- a total nitrogen content less than or equal to 6 g/Kg of biomass as such; and/or
- a value DM/Tq greater than or equal to 30% by weight, preferably greater than or equal to 35% by weight.

The value DM/Tq represents the quantity of dry matter relative to the overall weight of the biomass as such.

In one embodiment, the biomass has all of the above-described properties.

Preferably, the at least one bioreactor 2 consists of a plurality of bioreactors 2, preferably 25-45 bioreactors, wherein the total digestion volume is equal to the sum of the digestion volumes of each individual reactor. In this embodiment, each bioreactor is a wet bioreactor or a semi-dry bioreactor. In this embodiment, about 10-20% of the bioreactors are primary reactors and about 80-90% of the bioreactors are secondary reactors.

In step (a), the at least one bioreactor 2 preferably operates at temperatures of about 37°-50°C, that is, under mesophilic conditions or under thermophilic conditions.

In a preferred embodiment, the dwell time of the biomass in the at least one bioreactor 2 is about 18-24 hours.

Optionally, and preferably, step (a) can comprise a step of inoculating the biomass with a quantity of bacteria capable of bringing about the fermentation of the biomass under anaerobic conditions, such a quantity being sufficient to bring about the fermentation of the inoculated biomass. The bacteria used, as well as the quantity of inoculum, are of the type normally used in the sector and thus not further described here.

The operating conditions of the anaerobic digestion in the at least one bioreactor 2 are conditions normally used for the digestion of vegetable biomasses and, as such, can be easily determined by the person skilled in the art.

The product of the anaerobic digestion of the biomass is a flow 3 of biogas comprising methane and carbon dioxide and a flow 4 of digestate comprising a liquid phase and a solid phase.

In one embodiment, the flow 3 of biogas comprises about 45-70% by volume of methane, about 23-53% by volume of CO₂ and a quantity less than or equal to 2% by volume of further inevitable impurities, including H2S, H₂O and NH₃.

Step (b) of the process comprises sending the flow 3 of biogas to a biogas refining plant 5 to remove the CO₂, thus obtaining a flow 6 of CO₂ and a flow 7 of biomethane.

The refining plant 5 is located downstream of and in fluid connection with the bioreactor 2.

The flow 6 of CO₂ preferably comprises about 93.5-95.5% by volume of carbon dioxide, about 6.2-4.2% by volume of water (steam) and a quantity ≤ about 0.3% by volume of impurities comprising methane and hydrogen sulphide.

The flow 7 of biomethane preferably comprises a quantity ≥ 99.0% by volume of CH₄, preferably ≥ 99.9% by volume, and a quantity ≤ 1.0% by volume of non-condensable gas, optionally comprising a gas selected from among oxygen, nitrogen, hydrogen and mixtures thereof.

According to a particularly preferred variant, step (b) comprises:
(b1) removing the impurities other than CO₂ comprised in the flow 3 of biogas, supplying the flow 3 of biogas to at least a first plant for the removal of NH₃ and to at least a second plant for the removal of H₂S, thus obtaining a flow 3' of purified biogas; and
(b2) sending the flow 3' of purified biogas to the biogas refining plant 5 to selectively remove the CO₂ from said flow, thus obtaining a flow 6 of CO₂ and a flow 7 of biomethane.

The removal plants of step (b1) are not shown in figure 1.

The plant for the removal of hydrogen sulphide can be selected from among a biological desulphurisation system, an impregnated activated carbon system and a system for washing with alkalis (for example with sodium bicarbonate and/or sodium hydroxide) and combinations thereof. The plant for the removal of ammonia can be an activated carbon abatement system and/or a wet abatement system using acidic solutions (for example with sulphuric acid).

The above-described abatement systems are known in the sector and used for the same purposes, and are thus not further described here in detail.

Step (b1) can be carried out by removing H₂S and NH₃ from the flow 3 of biogas in any order, that is, by supplying the flow 3 of biogas first to at least a first plant for the removal of NH₃ and subsequently to at least a second plant for the removal of H₂S, or else supplying the flow 3 of biogas to said abatement systems in a reverse order.

The plant for the removal of ammonia and the plant for the removal of hydrogen sulphide are in fluid connection with each other, downstream and in fluid connection with the bioreactor 2, upstream and in fluid connection with the refining plant 5.

In a preferred embodiment the process comprises, after step b), or after step b2) when present, the further steps of:
(c) sending the flow 6 of CO₂ coming from the refining plant 5 to at least one methanation reactor 8 and converting the CO₂ at least partially into methane by reaction with hydrogen, thus obtaining a flow (10) of synthetic methane; and
(d) optionally, but preferably, mixing the flow 7 of biomethane coming from the refining plant 5 and the flow 10 of synthetic methane coming from the methanation reactor 8,
wherein the total quantity of methane produced is greater than or equal to 150,000,000 m³/year, preferably greater than or equal to 170,000,000 m³/year.

The at least one methanation reactor 8 is located downstream and in fluid connection with the refining plant 5.

According to this embodiment, the quantity of methane produced is extremely high, since the CO₂ comprised in the offgas produced by the refining plant 5 is converted into methane, thus increasing the methane yield of the raw materials and reducing the environmental impact of the entire process.

The CO₂ methanation reaction (Sabatier reaction) is preferably conducted at a temperature of 300°-400°C, optionally in the presence of a catalyst so as to increase the selectivity of the process and minimise the water gas shift reaction. The catalyst can be selected from among Ni, Ru, Rh, Fe and combinations thereof, preferably a silica-, alumina- or zeolite-supported catalyst.

Advantageously, the hydrogen used in the CO₂ methanation reaction can be produced by electrolysis of water in a hydrogen generator 11, thus generating a flow of oxygen 12 as a by-product.

The hydrogen generator 11 is in fluid connection with the methanation reactor 8.

The flow of oxygen 12 can optionally be stored for later use, thereby increasing the profitability of the entire process and minimising the gaseous emissions.

The process of the present description can preferably comprise at least one step of dehumidifying the biomethane of the flow 7 and/or the synthetic methane of the flow 10 in at least one dehumidification plant, not shown in figure 1, prior to the introduction of the flow 7 and/or of flow 10 into the network or storage in tanks.

Optionally, the methane produced by the process can be liquefied to enable a more efficient storage thereof.

The dehumidification plant and the liquefaction plant, not shown in the diagram in figure 1, are of a known type and used in the sector for the respective purposes.

Advantageously, the process of the present description comprises the use and exploitation of the digestate produced by the at least one bioreactor with the aim of optimising the economic returns of the process and minimising the environmental impact.

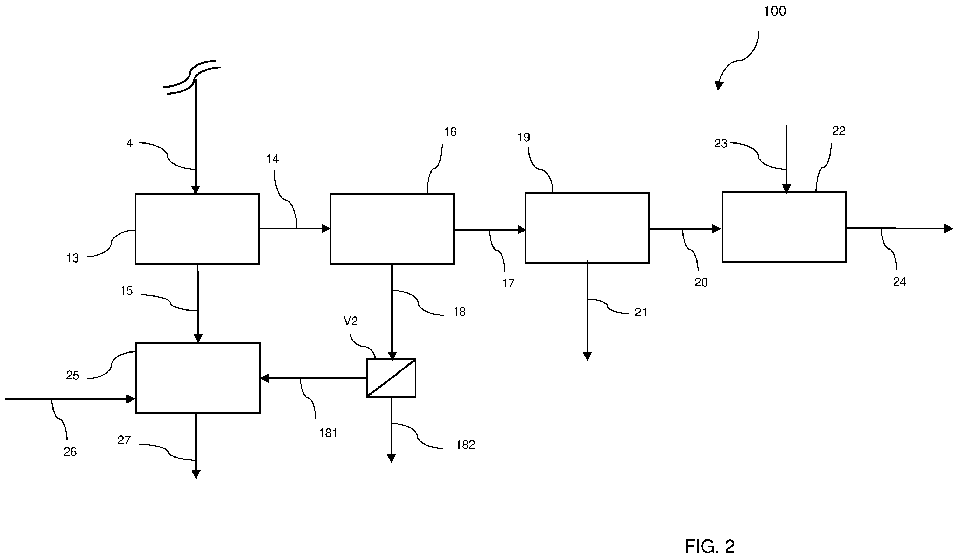

In a preferred embodiment, schematically reproduced in figure 2, the process comprises at least one step of separating the flow 4 of digestate in at least a first mechanical separation system 13, thus obtaining a solid phase 15 and a liquid phase 14, wherein the liquid phase 14 comprises water, mineral salts dissolved in water and dispersed solids.

Optionally, the process can comprise a step of storing the digestate in suitable storage tanks or settling tanks, upstream of the separation of the digestate carried out in the first separation system 13.

In one embodiment, the first mechanical separation system 13 is in fluid communication with the bioreactor 2.

Preferably, the first separation system 13 is a mechanical separation system capable of eliminating a large part of the solids comprised in the digestate. In one embodiment, the first mechanical separation system 13 comprises at least one helical separator in order to eliminate the coarse part of solids of the digestate followed downstream and at least one centrifuge placed in fluid communication with the helical separator. The solid phase 15 separated in the first separation system can be at least partially fed to a plant for the production of polyhydroxyalkanoates and/or disposed of. Said solid phase 15 can comprise up to 10% by weight of liquid, generally water, and manifest itself in the form of sludge, possibly shovelable.

The process preferably comprises a further step of treating the liquid phase 14 in a second separation system comprising at least one ultrafiltration plant 16 and at least one reverse osmosis separator 19, said reverse osmosis separator 19 being downstream of and in fluid communication with the ultrafiltration plant 16, said reverse osmosis separator 19 being further configured to obtain a saline concentrate 20 from the permeate 17 of the ultrafiltration plant, wherein a flow 20 of saline concentrate, a flow 18 comprising the dispersed solids and a flow 21 consisting essentially of water are obtained from the second separation system.

The ultrafiltration plant 16 and the reverse osmosis plant 19 are of the type normally used in the sector and thus not described here in detail.

In one embodiment, not represented in figure 2, the flow of retentate 18 comprising dispersed solids separated in the ultrafiltration plant is fed to at least one bioreactor 25 for the production of polyhydroxyalkanoates through fermentation. Optionally, the flow of retentate 18 can be sent to a separation device V2 configured to generate at least a first current of retentate 181 and/or at least a second current of retentate 182, wherein the first current of retentate 181 is supplied to the bioreactor 25. The concentration of solids in the first current 181 and/or in the second current 182 can be appropriately adjusted by using suitable concentrators.

In one variant of the process not represented in figure 2, the flow 18 of retentate comprising the dispersed solids can be at least partially recycled at the first separation system 13; in particular, it can be fed to the inlet of at least one centrifuge.

The process can further advantageously comprise a step of concentrating the mineral salts comprised in the saline concentrate 20 before supplying the mineral salts comprised in the concentrate to the fertiliser production plant. Subsequently, the process can comprise at least one step of mixing the mineral salts comprised in the saline concentrate 20, optionally appropriately concentrated, and optionally further fertilising elements 23 so as to produce a fertiliser 24 in a plant for the production of mineral fertilisers 22.

The plant for the production of mineral fertilisers 22 is a plant of the type known in the art, which comprises the following plant sections not represented in figure 2:
- at least one vat mixer;
- at least one granulator;
- at least one dryer, preferably a rotary drum dryer;
- at least one sieve connected to at least one bag filling machine.

The further fertilising elements 23 can be selected from among macroelements, that is, compounds containing nitrogen, phosphorous, potassium, calcium, magnesium, sulphur, chlorine, sodium and combinations thereof; and/or from among microelements, that is, compounds containing boron, manganese, copper, zinc, molybdenum, iron and combinations thereof.

In a preferred embodiment, the process further comprises the following steps:
- in a bioreactor (25), mixing the solid phase (15) and a bacterial biomass (26) able to synthesise polyhydroxyalkanoates; and
- fermenting the solid phase (15) by the bacterial biomass (26) and extracting the polyhydroxyalkanoates produced (27).

In particular, the process further comprises the following steps:
(i) in a bioreactor 25, mixing the solid phase 15 with the retentate selected from the retentate of the flow 18 and the retentate of the first current of retentate 181 and with a bacterial biomass 26 able to synthesise polyhydroxyalkanoates (PHA) from a source of carbon; and
(ii) fermenting the mixture obtained in step (i) by the bacterial biomass 26, extracting the polyhydroxyalkanoates produced 27.

Preferably, upstream of step (i), the solid phase 15 and/or the retentate 18 and/or the retentate of the first current of retentate 181 can be subjected to a high-temperature treatment in order to reduce the carbon that cannot be digested by the PHA-producing bacteria and transform it into a more useful substrate for the production of PHA. This treatment can take place in a plant not schematically illustrated in figure 2, known in the art, operating for example at a temperature of 160°-180°C and at a pressure of 16-18 bar.

In a further aspect thereof, the present description relates to a plant 100 for producing methane from the anaerobic fermentation of a biomass, schematically represented in figure 1, the plant 100 comprising:
- at least one bioreactor 2 for the production of biogas from the anaerobic fermentation of a biomass, said bioreactor 2 having a total digestion volume less than or equal to 350,000 m³, preferably 150,000-350,000 m³, which is supplied with a biomass 1 and from which at least a first flow 3 of biogas exits, said flow (3) comprising methane and CO₂ and at least a flow of digestate 4 comprising a solid phase and a liquid phase;
- at least one separation device V1 connected to the bioreactor 2, said separation device V1 receiving the flow 3 of biogas and being configured to separate the flow 3 of biogas into a first current 31 of biogas and/or a second current 32 of biogas;
- at least one removal plant 5 adapted to remove the CO₂ from the flow of biogas 3 and/or from the flow of biogas 31, said removal plant 5 being configured to receive the flow 3 of biogas and/or the flow of biogas 31, wherein at least one flow 6 of CO₂ and at least one flow 7 of methane gas exit from the removal plant 5;
- at least one methanation reactor 8 for converting the CO₂ into methane by reaction of the CO₂ with hydrogen, said methanation reactor 8 being configured to receive the flow 6 of CO₂ coming from the CO₂ removal system 5 and the second current 32 of biogas and at least one flow 9 of hydrogen, wherein at least one flow 10 of methane gas exits from the methanation reactor 8;
- a connection means 71 for connecting the plant 100 with the methane gas distribution network and/or with at least one storage tank.

In the plant described above, the CO₂ removal plant 5 is placed downstream of and in fluid communication with the bioreactor 2, optionally downstream of and in fluid communication with the separator V1. The methanation reactor 8 is placed downstream of the CO₂ removal plant 5 and is placed in fluid communication therewith.

The methanation reactor 8, furthermore, is in fluid communication with the bioreactor 2, optionally through the separation device V1.

In one embodiment, the flow 10 of methane gas coming from the methanation reactor 8 is connected to the flow 7 of methane gas coming from the CO₂ removal system 5.

Preferably, the plant 100 further comprising at least one hydrogen generator 11 in fluid connection with the methanation reactor 8, said hydrogen generator 11 being configured to produce the hydrogen comprised in the flow 9 from the hydrolysis of water, generating a flow of oxygen 12 as a by-product.

In one embodiment, the plant 100 further comprises:
- at least one separation device 13 for separating the liquid 14 and solid 15 phases of the digestate, said separation system 13 being supplied with the digestate 4 and being configured to separate the digestate into a liquid fraction 14 comprising water, mineral salts dissolved in water and dispersed solids, and a solid fraction 15;
- at least a first filtration system 16 for separating the suspended solids comprised in the liquid fraction of the digestate, said first filtration system 16 being connected to the mechanical separation system 13 and supplied with the liquid fraction 14 and being configured to separate the liquid fraction 14 into a flow 17 of permeate comprising dissolved salts and into a flow 18 of retentate;
- at least one separation device V2 connected to the first filtration system 16 and configured to receive the flow 18 of retentate, said separation device V2 being configured to selectively separate the retentate 18 into a first current of retentate 181 and/or into a second current of retentate 182;
- at least a second filtration system 19 for the concentration of the salts dissolved in the flow of permeate, said second filtration system 19 being placed downstream of and in fluid communication with the first filtration system 16 and being supplied with the flow 17 of permeate, wherein said second filtration system 19 is configured to generate a flow 20 of saline concentrate and a flow 21 of water;
- a plant 22 for the production of fertiliser configured to use the saline concentrate obtained from the second filtration system 20 in the production of mineral fertilisers, said plant 22 for the production of fertiliser comprising at least one system for supplying the flow 20 of saline concentrate and at least one system 23 for supplying mineral fertilising elements and generating at least one outlet flow 24 of the fertiliser.

The separation system 13 is preferably a mechanical separation system as described above.

The first filtration system 16 is preferably an ultrafiltration plant and the second filtration system 19 is preferably a reverse osmosis separator.

In a further embodiment, the plant for producing methane to which the present description relates further comprises a plant section 25 for producing polyhydroxyalkanoates from the bacterial fermentation of the solid phase of the digestate, said plant 25 comprising a means for supplying a bacterial biomass 26 able to produce polyhydroxyalkanoates, a means for supplying the solid fraction 15 obtained from the mechanical separation of the digestate 4 and a means for supplying the current of retentate 182 obtained from the first filtration system 16, and wherein said plant 25 is configured to produce a flow 27 comprising polyhydroxyalkanoates.

The plant for producing methane described above is configured to produce a quantity of methane gas greater than or equal to 70,000,000 m³/year, preferably greater than or equal to 90,000,000 m³/year, more preferably greater than or equal to 150,000,000 m³/year, more preferably greater than or equal to 170,000,000 m³/year from the anaerobic fermentation of a quantity of biomass 1 greater than or equal to 700,000 Kg/year, preferably 700,000-900,000 Kg/year.

## Claims

1. A plant for producing methane from the anaerobic fermentation of a biomass, the plant (100) comprising:
- at least one bioreactor (2) for the production of biogas from the anaerobic fermentation of a biomass, said bioreactor (2) having a total digestion volume less than or equal to 350,000 m³, preferably 150,000-350,000 m³, which is supplied with a biomass (1) and from which at least a first flow (3) of biogas exits, said flow (3) comprising methane and CO₂ and a digestate (4) comprising a solid phase and a liquid phase;
- at least one separation device (V1) connected to the bioreactor (2), said separation device (V1) receiving the flow (3) of biogas and being configured to separate the flow (3) of biogas into a first current (31) of biogas and/or a second current (32) of biogas;
- at least one removal plant (5) adapted to remove the CO₂ from the flow of biogas (3) and/or from the flow of biogas (31), said CO₂ removal plant (5) being configured to receive the flow (3) of biogas and/or the flow of biogas (31), wherein at least one flow (6) of CO₂ and at least one flow (7) of methane gas exit from the removal plant (5);
- at least one methanation reactor (8) for converting the CO₂ into methane by reaction of CO₂ with hydrogen, said methanation reactor (8) being configured to receive at least one flow (9) of hydrogen, the flow (6) of CO₂ coming from the CO₂ removal system (5) and the second current (32) of biogas, and wherein at least one flow (10) of methane gas exits from the methanation reactor (8);
- a connection means (71) for connecting the plant (100) with the methane gas distribution network and/or with at least one storage tank.

2. The plant for producing methane according to claim 1, wherein the flow (10) of methane gas coming from the methanation reactor (8) is connected to the flow (7) of methane gas coming from the CO₂ removal system (5).

3. The plant for producing methane according to any one of claims 1 or 2, further comprising at least one hydrogen generator (11) connected to the methanation reactor (8), said hydrogen generator (11) being configured to produce the hydrogen comprised in the flow (9) from the hydrolysis of water, generating a flow of oxygen (12) as a by-product.

4. The plant for producing methane according to any one of claims 1-3, further comprising:
- at least one mechanical separation device (13) for separating the liquid and solid phases of the digestate, said mechanical separation system (13) being supplied with the digestate (4) and being configured to separate the digestate into a liquid fraction (14) comprising suspended solids and a solid fraction (15);
- at least a first filtration system (16) for separating the suspended solids comprised in the liquid fraction of the digestate, said first filtration system (16) being connected to the mechanical separation system (13) and supplied with the liquid fraction (14) and being configured to separate the liquid fraction (14) into a flow (17) of permeate comprising dissolved salts and a retentate (18);
- at least one separation device (V2) connected to the first filtration system (16) and configured to receive the retentate (18), said separation device (V2) being configured to selectively separate the retentate (18) into a first current of retentate (181) and/or into a second current of retentate (182);
- at least a second filtration system (19) for the concentration of the salts dissolved in the flow of permeate, said second filtration system (19) being placed downstream of and in fluid communication with the first filtration system (16) and being supplied with the flow (17) of permeate, wherein said second filtration system (19) is configured to generate a flow (20) of saline concentrate and a flow (21) of water;
- a plant (22) for the production of fertiliser configured to use the saline concentrate obtained from the second filtration system (20) in the production of mineral fertilisers, said plant (22) for the production of fertiliser comprising at least one system for supplying the flow (20) of saline concentrate, at least one system (23) for supplying mineral fertilising elements and generating at least one outlet flow (24) of the fertiliser.

5. The plant for producing methane according to any one of claims1-4, further comprising a plant (25) for producing polyhydroxyalkanoates from the bacterial fermentation of the solid phase of the digestate, said plant (25) comprising a means for supplying a bacterial biomass (26) able to produce polyhydroxyalkanoates, a means for supplying the solid fraction (15) obtained from the mechanical separation of the digestate (4) and a means for supplying the current of retentate (182) obtained from the first filtration system (16) and wherein said plant (25) is configured to produce a flow (27) comprising polyhydroxyalkanoates.

6. The plant for producing methane according to any one of claims 1-5, configured to produce a quantity of methane gas greater than or equal to 70,000,000 m³/year, preferably greater than or equal to 90,000,000 m³/year, more preferably greater than or equal to 150,000,000 m³/year, preferably greater than or equal to 170,000,000 m³/year from the anaerobic fermentation of a quantity of biomass (1) greater than or equal to 700,000 Kg/year, preferably 700,000-900,000 Kg/year.

7. A process for producing methane from the anaerobic fermentation of a biomass, the process comprising at least the following steps:
(a) subjecting to anaerobic digestion a quantity greater than or equal to 700,000 t/year, preferably 700,000-900,000 t/year, of biomass as such, having an average value VS/Tq greater than or equal to 27.0% by weight, preferably greater than or equal to 30.0% by weight, more preferably greater than or equal to 33.0% by weight, in at least one bioreactor (2) having a total digestion volume less than or equal to 350,000 m³, preferably 150,000-350,000 m³, and producing a flow (3) of biogas comprising methane and CO₂ and a flow (4) of digestate comprising a solid phase and a liquid phase;
(b) sending the flow (3) of biogas to a biogas refining plant (5) to selectively remove the CO₂ from the flow (3) of biogas, thus obtaining a flow (6) of CO₂ and a flow (7) of biomethane;
wherein the quantity of methane produced is greater than or equal to 70,000,000 m³/year, preferably greater than or equal to 90,000,000 m³/year.

8. The process according to claim 7, wherein the biomass as such is a vegetable biomass, preferably comprising waste and/or by-products of the production of sugar from sugar beet.

9. The process according to any one of the preceding claims, wherein the biomass as such comprises:
(A) 20-30% by weight of sugar beet pulp;
(B) 15-30% by weight of molasses;
(C) 50-60% by weight of sugar beet leaves and sugar beet tops,
wherein the quantities (A), (B) and (C) refer to the total weight of (A)+(B)+(C).

10. The process according to any one of the preceding claims, wherein the biomass as such has at least one of the following properties:
- total nitrogen content less than or equal to 6 g/Kg of biomass as such; and/or
- average value DM/Tq greater than or equal to 30% by weight, preferably greater than or equal to 35% by weight.

11. The process according to any one of the preceding claims, comprising after step b) the steps of:
(c) sending the flow (6) of CO₂ coming from the refining plant (5) to at least one methanation reactor (8) and converting the CO₂ into methane by reaction with hydrogen, thus obtaining a flow (10) of synthetic methane; and
(d) optionally, but preferably, mixing the flow (7) of biomethane coming from the refining plant (5) and the flow (10) of synthetic methane coming from the methanation reactor (8),
wherein the total quantity of methane produced is greater than or equal to 150,000,000 m³/year, preferably greater than or equal to 170,000,000 m³/year.

12. The process according to claim 11, wherein the hydrogen is produced by the electrolysis of water in a hydrogen generator (11), generating a flow of oxygen (12) as a by-product.

13. The process according to any one of the preceding claims, further comprising at least one step of separating the flow (4) of digestate in at least a first mechanical separation system (13), thus obtaining a solid phase (15) and a liquid phase (14), wherein the liquid phase (14) comprises water, mineral salts dissolved in water and dispersed solids.

14. The process according to claim 13, further comprising at least a step of treating the liquid phase (14) in a second separation system so as to obtain a flow (20) of saline concentrate, a flow (18) comprising the dispersed solids and a flow (21) of water, wherein the separation system comprises at least one ultrafiltration plant (16) and at least one reverse osmosis separator (19) downstream of the ultrafiltration plant (16).

15. The process according to claim 13 or 14, further comprising at least one step of mixing the mineral salts comprised in the aqueous composition (20) and, optionally, further fertilising elements (23) so as to produce a fertiliser (24).
